# EUROPEAN PATENT APPLICATION

(11) **EP 4 847 773 A1**
(43) Date of publication of application: **02.12.2026**
(21) Application number: 25777996.7
(22) Date of filing: 27.02.2025
(51) Int. Cl.: F04B 49/20, A61M 1/06, F04B 49/06

(54) **ADAPTIVE METHOD AND APPARATUS FOR OPERATING FREQUENCY OF BREAST PUMP, BREAST PUMP, AND STORAGE MEDIUM**

(30) Priority: 29.03.2024 CN 202410379821
(71) Applicant: Shenzhen Root Innovation Technology Industry Co., Ltd., Shenzhen, Guangdong 518000 (CN)
(72) Inventor: WANG, Dandan, Shenzhen, Guangdong 518000 (CN); CHEN, Xiaozhuan, Shenzhen, Guangdong 518000 (CN)
(74) Representative: Winter, Brandl - Partnerschaft mbB
(86) International application number: PCT/CN2025/079532
(87) International publication number: WO 2025/200922

(57) **Abstract**

An adaptive method for the operating frequency of a breast pump. The adaptive method comprises: within a first time period after a piezoelectric pump of a breast pump is started, adjusting the operating frequency of the piezoelectric pump, and testing suction output capabilities of the piezoelectric pump at different operating frequencies; acquiring a corresponding operating frequency at the maximum output capability of the piezoelectric pump, and using the corresponding operating frequency at the maximum output capability of the piezoelectric pump as an optimal operating frequency within the current time period; and within a second time period following the first time period, setting the operating frequency of the piezoelectric pump to be the optimal operating frequency, and maintaining the optimal operating frequency for operation. The method enables a piezoelectric pump to maintain operation in an optimal state, thereby improving the output power and efficiency of the piezoelectric pump of a breast pump, and reducing the power loss.

## Description

### RELATED APPLICATIONS

The present application claims priority to Chinese Patent Application No. 202410379821.4 filed on March 29, 2024, the entire contents of which are incorporated herein by reference.

### TECHNICAL FIELD

The embodiments of the present disclosure relate to the technical field of maternal and infant devices, and in particular, to an adaptive method and apparatus for the operating frequency of a breast pump, a breast pump, and a storage medium.

### BACKGROUND

A breast pump is an auxiliary tool configured to extract breast milk accumulated in mammary glands. Common breast pumps are single-sided electric or double-sided electric types. Breast pumps help unblock mammary glands and promptly empty breast milk. Breast pumps generally use built-in vacuum pumps and solenoid valves. For portability and convenience, rechargeable batteries are usually built in as well.

With technological advancements, breast pumps have gradually evolved from split-type to integrated wearable types. The volume of wearable breast pumps is also gradually developing in a miniaturized direction. A piezoelectric pump is a small-volume, low-noise negative pressure pump, and is an important component that cannot be avoided in the miniaturized design of breast pumps. Due to the characteristics of piezoelectric pumps, the optimal operating frequency is different for each piezoelectric pump. If a piezoelectric pump does not operate at its optimal operating frequency, it leads to high energy consumption and low suction force, greatly reducing the output power and efficiency of the piezoelectric pump. Therefore, how to make a piezoelectric pump operate at its optimal frequency is an important issue.

### SUMMARY OF THE DISCLOSURE

In view of the deficiencies existing in the related art, the present disclosure provides a control method and apparatus for a breast pump, a breast pump, and a storage medium, which may solve the problem of low output power and efficiency of the piezoelectric pump in a breast pump.

To achieve the above objective, a technical solution adopted by the present disclosure is: providing an adaptive method for an operating frequency of a breast pump, the adaptive method including:
adjusting an operating frequency of a piezoelectric pump of the breast pump within a first time period after the piezoelectric pump is started, and detecting suction output capabilities of the piezoelectric pump at different operating frequencies;
obtaining the operating frequency corresponding to a maximum output capability of the piezoelectric pump and taking the operating frequency corresponding to the maximum output capability as an optimal operating frequency for a current time period; and
setting the operating frequency of the piezoelectric pump to the optimal operating frequency and maintaining operation at the optimal operating frequency within a second time period after the first time period.

In some embodiments, the detecting suction output capabilities of the piezoelectric pump at different operating frequencies includes:
detecting suction values output by the piezoelectric pump at different operating frequencies;
the obtaining the operating frequency corresponding to a maximum output capability of the piezoelectric pump and taking the operating frequency corresponding to the maximum output capability as an optimal operating frequency for a current time period includes:
   obtaining the operating frequency corresponding to a maximum suction value of the piezoelectric pump and taking the operating frequency corresponding to the maximum suction value as the optimal operating frequency for the current time period.

In some embodiments, the detecting the suction output capabilities of the piezoelectric pump at different operating frequencies includes:
detecting working current values of the piezoelectric pump at different operating frequencies;
the obtaining the operating frequency corresponding to a maximum output capability of the piezoelectric pump and taking the operating frequency corresponding to the maximum output capability as an optimal operating frequency for a current time period includes:
   obtaining the operating frequency corresponding to a maximum working current value of the piezoelectric pump and taking the operating frequency corresponding to the maximum working current value as the optimal operating frequency for the current time period.

In some embodiments, the detecting the suction output capabilities of the piezoelectric pump at different operating frequencies includes:
detecting working voltage values of the piezoelectric pump at different operating frequencies;
the obtaining the operating frequency corresponding to a maximum output capability of the piezoelectric pump and taking the operating frequency corresponding to the maximum output capability as an optimal operating frequency for a current time period includes:
   obtaining the operating frequency corresponding to a maximum working voltage value of the piezoelectric pump and taking the operating frequency corresponding to the maximum working voltage value as the optimal operating frequency for the current time period.

In some embodiments, the adaptive method further includes:
readjusting the operating frequency of the piezoelectric pump within a third time period after the second time period, and detecting the suction output capabilities of the piezoelectric pump at different operating frequencies;
obtaining the operating frequency corresponding to another maximum output capability of the piezoelectric pump and taking the operating frequency corresponding to the other maximum output capability as another optimal operating frequency for another current time period; and
setting the operating frequency of the piezoelectric pump to the other optimal operating frequency and maintaining operation at the other optimal operating frequency within a fourth time period after the third time period.

In some embodiments, the adjusting an operating frequency of a piezoelectric pump of the breast pump within a first time period after the piezoelectric pump is started, and the detecting suction output capabilities of the piezoelectric pump at different operating frequencies include:
adjusting the operating frequency of the piezoelectric pump within a preset frequency range within the first time period after the piezoelectric pump is started, and detecting the suction output capabilities of the piezoelectric pump at different operating frequencies.

In some embodiments, the adaptive method further includes:
detecting the operating frequency of the piezoelectric pump during operation of the breast pump, and determining a maximum value and a minimum value of the operating frequency; and
determining the preset frequency range according to the maximum value and the minimum value.

To achieve the above objective, another technical solution adopted by the present disclosure is: providing a breast pump, the breast pump including a processor and a memory electrically connected to the processor, the memory storing a computer program, and the processor being configured to invoke the computer program to execute the above adaptive method for the operating frequency of a breast pump.

To achieve the above objective, yet another technical solution adopted by the present disclosure is: providing a storage medium, the storage medium storing a computer program, and the computer program being capable of being executed to implement the above adaptive method for the operating frequency of a breast pump.

To achieve the above objective, still another technical solution adopted by the present disclosure is: providing an adaptive apparatus for the operating frequency of a breast pump, the adaptive apparatus including:
an adjustment and detection module, configured to adjust an operating frequency of a piezoelectric pump of the breast pump within a first time period after the piezoelectric pump is started, and detect suction output capabilities of the piezoelectric pump at different operating frequencies;
an acquisition module, configured to obtain the operating frequency corresponding to a maximum output capability of the piezoelectric pump and take the operating frequency corresponding to the maximum output capability as an optimal operating frequency for a current time period; and
a setting module, configured to set the operating frequency of the piezoelectric pump to the optimal operating frequency and maintaining operation at the optimal operating frequency within a second time period after the first time period.

To achieve the above objective, a further technical solution adopted by the present disclosure is: providing a breast pump, the breast pump including a processor and a sensor electrically connected to the processor, the processor being configured to adjust the operating frequency of a piezoelectric pump of the breast pump within a first time period after the piezoelectric pump is started, and control the sensor to detect a suction output capability of the piezoelectric pump at different operating frequencies; the processor being further configured to obtain the operating frequency corresponding to a maximum output capability of the piezoelectric pump and use the operating frequency corresponding to the maximum output capability as an optimal operating frequency for a current time period; the processor being further configured to set the operating frequency of the piezoelectric pump to the optimal operating frequency and maintain operation at the optimal operating frequency during a second time period after the first time period.

The embodiments of the present disclosure, by adjusting the operating frequency of the piezoelectric pump within the first time period after the piezoelectric pump is started, and detecting the suction output capabilities of the piezoelectric pump at different operating frequencies; obtaining the operating frequency corresponding to the maximum output capability of the piezoelectric pump and taking the operating frequency corresponding to the maximum output capability as the optimal operating frequency for the current time period; and setting the operating frequency of the piezoelectric pump to the optimal operating frequency and maintaining operation at the optimal operating frequency during the second time period after the first time period, may make the piezoelectric pump operate in an optimal state, thereby improving the output power and efficiency of the breast pump's piezoelectric pump.

### BRIEF DESCRIPTION OF THE DRAWINGS

In order to explain the technical solutions in the embodiments of the present disclosure or the related art more clearly, the following will briefly introduce the drawings required for describing the embodiments or the related art. Obviously, the drawings in the following description are only some embodiments of the present disclosure. For those skilled in the art, other drawings may be obtained based on the structures shown in these drawings without creative effort.
FIG. 1 is a schematic flowchart of an adaptive method for an operating frequency of a breast pump according to a first embodiment of the present disclosure.
FIG. 2 is a schematic flowchart of an adaptive method for an operating frequency of a breast pump according to a second embodiment of the present disclosure.
FIG. 3 is a schematic flowchart of an adaptive method for an operating frequency of a breast pump according to a third embodiment of the present disclosure.
FIG. 4 is a schematic flowchart of an adaptive method for an operating frequency of a breast pump according to a fourth embodiment of the present disclosure.
FIG. 5 is a schematic block diagram of an adaptive apparatus for an operating frequency of a breast pump according to some embodiments of the present disclosure.
FIG. 6 is a schematic diagram of a hardware structure of a breast pump according to some embodiments of the present disclosure.
FIG. 7 is a schematic diagram of another hardware structure of a breast pump according to some embodiments of the present disclosure.

The realization of the objectives, functional features, and advantages of the present disclosure will be further described in conjunction with the embodiments and with reference to the accompanying drawings.

### DETAILED DESCRIPTION

In the present disclosure, the terms "arranged with", "provided with", and "connected" should be understood broadly. For example, the connection may be a fixed connection, a detachable connection, or an integral structure; it may be a mechanical connection or an electrical connection; it may be a direct connection, an indirect connection through an intermediary, or internal communication between two devices, components, or constituent parts. For those skilled in the art, the specific meanings of the above terms in the present disclosure may be understood according to specific circumstances.

The orientation or positional relationships indicated by terms such as "center", "longitudinal", "transverse", "length", "width", "thickness", "upper", "lower", "front", "rear", "left", "right", "vertical", "horizontal", "top", "bottom", "inner", "outer", "radial", "circumferential", etc. are based on the orientation or positional relationships shown in the drawings, and are only for the convenience of describing the present disclosure and simplifying the description, rather than indicating or implying that the device or element referred to must have a specific orientation, be constructed and operate in a specific orientation, and therefore cannot be construed as limitations to the present disclosure.

Furthermore, the terms "first" and "second" are for descriptive purposes only and are not to be construed as indicating or implying relative importance or implicitly indicating the number of technical features indicated. Thus, features defined as "first" or "second" may explicitly or implicitly include at least one such feature. In the description of the present disclosure, "a plurality" means at least two, such as two, three, etc., unless otherwise specifically defined.

Moreover, in addition to being intended to indicate orientation or positional relationships, the aforementioned terms may further be configured to indicate other meanings. For example, the term "upper" may in some cases be configured to indicate a certain attachment or connection relationship. For those skilled in the art, the specific meanings of these terms in the present disclosure may be understood according to specific circumstances.

To make the objectives, technical solutions, and advantages of the present disclosure clearer, the present disclosure will be further described in detail below with reference to the accompanying drawings and embodiments. It should be understood that the specific embodiments described herein are only intended to explain the present disclosure and are not to limit the present disclosure.

A piezoelectric pump is a type of fluid driver. It does not require an additional drive motor. Instead, it utilizes the inverse piezoelectric effect of piezoelectric material to cause the piezoelectric vibrator to deform, and then uses the deformation to change the volume of the pump chamber to achieve fluid output, or uses the vibrator to generate waves to drive the fluid and create negative pressure. Piezoelectric pumps are small in size and have low noise. However, there are also some problems. For example, the inherent frequency of the medium is different for each piezoelectric pump, and the consistency cannot be guaranteed during manufacturing, meaning the inherent frequencies of the media cannot be completely identical. Thus, the resonant frequency of the medium is also different for each piezoelectric pump when it leaves the factory. In other words, to achieve the best output performance of the piezoelectric pump, it is necessary to find this optimal operating frequency. Another problem is that as the piezoelectric pump operates for a longer time and its temperature increases, the resonant frequency of the medium also changes, and accordingly, its optimal operating frequency also changes. For example, a common piezoelectric pump uses a ceramic medium, i.e., a piezoelectric ceramic pump. However, the piezoelectric pump mentioned in the embodiments of the present disclosure is not limited to piezoelectric ceramic pumps; piezoelectric pumps made of other materials are also applicable, which is not limited in the present disclosure.

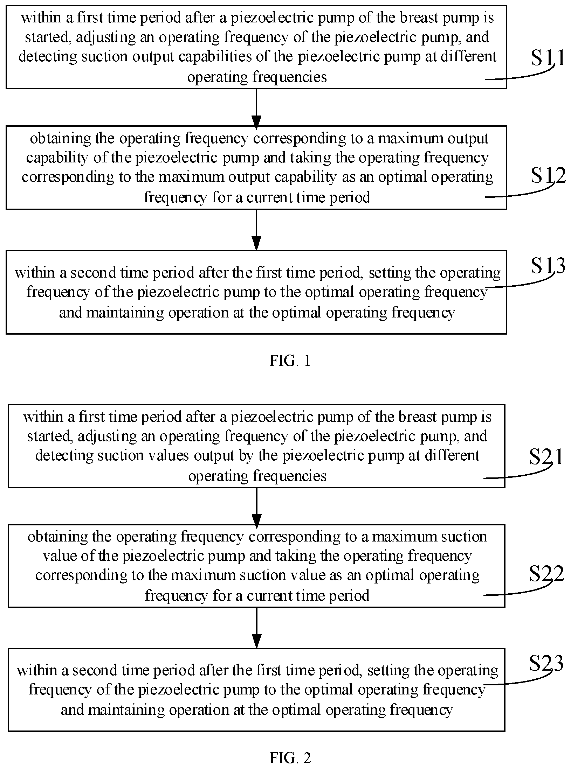

Referring to FIG. 1, FIG. 1 is a schematic flowchart of an adaptive method for an operating frequency of a breast pump according to a first embodiment of the present disclosure.

In the embodiments, the adaptive method for an operating frequency of a breast pump may include the following operations.

At block S11: within a first time period after a piezoelectric pump of the breast pump is started, adjusting an operating frequency of the piezoelectric pump, and detecting suction output capabilities of the piezoelectric pump at different operating frequencies.

For example, the first time period may be within 5 seconds after startup. Within the initial 5 seconds after the piezoelectric pump of the breast pump is started, the operating frequency of the piezoelectric pump is changed, for example: varying the operating frequency between 18 KHz and 24 KHz, changing by 0.01 KHz each time, collecting 600 data points, and performing corresponding detection each time the operating frequency changes to detect the suction output capabilities of the piezoelectric pump at different operating frequencies. The suction output capability of the piezoelectric pump may be output power, working current, working voltage, output suction value, etc., of the piezoelectric pump. A correspondence table between operating frequency and suction output capability is established, and the detected data associated with the corresponding operating frequency is stored.

At block S12: obtaining the operating frequency corresponding to a maximum output capability of the piezoelectric pump and taking the operating frequency corresponding to the maximum output capability as an optimal operating frequency for a current time period.

For example, the processor of the breast pump, by comparing and analyzing the values of the suction output capabilities, looks up the operating frequency corresponding to the maximum value of the suction output capability in the correspondence table between operating frequency and suction output capability. The suction output capability may be a parameter reflecting the output capability of the breast pump, such as the output suction value of the piezoelectric pump, the working current of the piezoelectric pump, the working voltage of the piezoelectric pump, etc.

At block S13: within a second time period after the first time period, setting the operating frequency of the piezoelectric pump to the optimal operating frequency and maintaining operation at the optimal operating frequency.

For example, the maintaining the operating frequency may be specifically achieved through a frequency comparison unit. The reference frequency of the frequency comparison unit may be set to the optimal operating frequency. The frequency comparison unit compares the phase of the reference frequency and the operating frequency of the piezoelectric pump to make the two frequencies consistent, thereby keeping the frequency of the piezoelectric pump at the optimal operating frequency. Specifically, the frequency comparison unit may be implemented using a phase-locked loop.

The embodiments of the present disclosure, by adjusting the operating frequency of the piezoelectric pump within the first time period after the piezoelectric pump is started, and detecting the suction output capabilities of the piezoelectric pump at different operating frequencies; obtaining the operating frequency corresponding to the maximum output capability of the piezoelectric pump and taking the operating frequency corresponding to the maximum output capability as the optimal operating frequency for the current time period; and setting the operating frequency of the piezoelectric pump to the optimal operating frequency and maintaining operation at the optimal operating frequency during the second time period after the first time period, may make the piezoelectric pump operate in an optimal state, thereby improving the output power and efficiency of the breast pump's piezoelectric pump.

Referring to FIG. 2, FIG. 2 is a schematic flowchart of an adaptive method for an operating frequency of a breast pump according to a second embodiment of the present disclosure.

In the embodiments, the detecting the suction output capabilities of the piezoelectric pump at different operating frequencies includes: detecting suction values output by the piezoelectric pump at different operating frequencies. The obtaining the operating frequency corresponding to the maximum output capability of the piezoelectric pump and taking the operating frequency corresponding to the maximum output capability as the optimal operating frequency for the current time period includes: obtaining the operating frequency corresponding to a maximum suction value of the piezoelectric pump and taking the operating frequency corresponding to the maximum suction value as the optimal operating frequency for the current time period.

In the embodiments, the adaptive method for an operating frequency of a breast pump may include the following operations.

At block S21: within a first time period after a piezoelectric pump of the breast pump is started, adjusting an operating frequency of the piezoelectric pump, and detecting suction values output by the piezoelectric pump at different operating frequencies.

After the piezoelectric pump of the breast pump is started, it first enters a specific time period called the "first time period". Within this time period, the system adjusts the operating frequency of the piezoelectric pump. The operating frequency refers to the number of working cycles or vibrations of the piezoelectric pump per unit time. Simultaneously, the system detects the output suction values of the piezoelectric pump at different operating frequencies. The suction value reflects the magnitude of the suction force generated by the piezoelectric pump, which is measured by a pressure sensor. The pressure sensor may be placed at any position on the output pipeline of the piezoelectric pump to accurately measure the suction force.

At block S22: obtaining the operating frequency corresponding to a maximum suction value of the piezoelectric pump and taking the operating frequency corresponding to the maximum suction value as an optimal operating frequency for a current time period.

The breast pump records and analyzes all suction values detected during the first time period and their corresponding operating frequencies. The purpose is to find the operating frequency corresponding to the maximum suction value. This frequency is considered the "optimal operating frequency" of the piezoelectric pump for the current time period. Once the optimal operating frequency is determined, the system saves it for subsequent use.

The suction value of the piezoelectric pump may be detected using a pressure sensor. The pressure sensor may be placed at any position on the output pipeline of the piezoelectric pump.

At block S23: within a second time period after the first time period, setting the operating frequency of the piezoelectric pump to the optimal operating frequency and maintaining operation at the optimal operating frequency.

After the first time period ends, it enters a second time period. Within this time period, the system sets the operating frequency of the piezoelectric pump to the previously determined optimal operating frequency. The piezoelectric pump will continue to operate at this optimal operating frequency to provide the maximum suction value, thereby ensuring the efficiency and performance of the breast pump.

In one implementation, a phase-locked loop may be arranged to keep the operating frequency of the piezoelectric pump at the optimal operating frequency. The phase-locked loop includes: 1. A voltage-controlled oscillator (VCO) for generating and outputting a drive signal related to the set target frequency to the piezoelectric pump. 2. A phase-frequency detector (PFD) for receiving the actual operating frequency signal of the piezoelectric pump and comparing it with the set optimal operating frequency signal to generate an error signal. 4. A low-pass filter (LPF) and a controller, which process the received error signal and then adjust the input voltage of the VCO, thereby changing the output frequency of the VCO. This phase-locked loop forms a closed-loop control system. Under the action of the phase-locked loop, the operating frequency of the piezoelectric pump automatically tracks and eventually locks onto the preset optimal operating frequency. Specifically, it may include the following steps: setting the target operating frequency of the phase-locked loop to the optimal operating frequency. The PFD calculates the error between the actual frequency and the target frequency and passes it to the LPF and the controller. The controller adjusts the output frequency of the VCO according to the error signal until the actual frequency matches the set optimal operating frequency, achieving a locked state. In the locked state, the system continuously monitors and dynamically fine-tunes to keep the operating frequency of the piezoelectric pump precisely locked to the set optimal operating frequency.

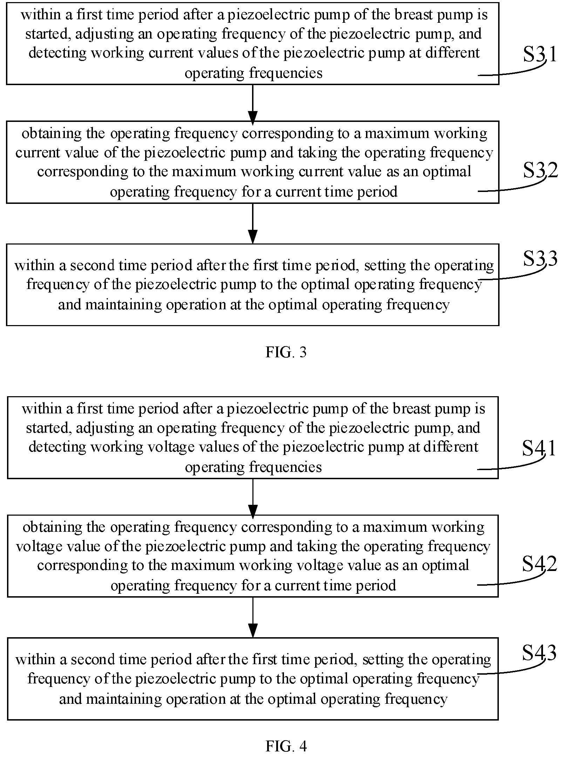

Referring to FIG. 3, FIG. 3 is a schematic flowchart of an adaptive method for an operating frequency of a breast pump according to a third embodiment of the present disclosure.

In the embodiments, the detecting the suction output capabilities of the piezoelectric pump at different operating frequencies includes: detecting working current values of the piezoelectric pump at different operating frequencies. The obtaining the operating frequency corresponding to the maximum output capability of the piezoelectric pump and taking the operating frequency corresponding to the maximum output capability as the optimal operating frequency for the current time period includes: obtaining the operating frequency corresponding to a maximum working current value of the piezoelectric pump and taking the operating frequency corresponding to the maximum working current value as the optimal operating frequency for the current time period.

In the embodiments, the adaptive method for an operating frequency of a breast pump may include the following operations.

At block S31: within a first time period after a piezoelectric pump of the breast pump is started, adjusting an operating frequency of the piezoelectric pump, and detecting working current values of the piezoelectric pump at different operating frequencies.

At block S32: obtaining the operating frequency corresponding to a maximum working current value of the piezoelectric pump and taking the operating frequency corresponding to the maximum working current value as an optimal operating frequency for a current time period.

At block S33: within a second time period after the first time period, setting the operating frequency of the piezoelectric pump to the optimal operating frequency and maintaining operation at the optimal operating frequency.

Referring to FIG. 4, FIG. 4 is a schematic flowchart of an adaptive method for an operating frequency of a breast pump according to a fourth embodiment of the present disclosure.

In the embodiments, the detecting the suction output capabilities of the piezoelectric pump at different operating frequencies includes: detecting working voltage values of the piezoelectric pump at different operating frequencies. The obtaining the operating frequency corresponding to the maximum output capability of the piezoelectric pump and taking the operating frequency corresponding to the maximum output capability as the optimal operating frequency for the current time period includes: obtaining the operating frequency corresponding to a maximum working voltage value of the piezoelectric pump and taking the operating frequency corresponding to the maximum working voltage value as the optimal operating frequency for the current time period.

In the embodiments, the adaptive method for an operating frequency of a breast pump may include the following operations.

At block S41: within a first time period after a piezoelectric pump of the breast pump is started, adjusting an operating frequency of the piezoelectric pump, and detecting working voltage values of the piezoelectric pump at different operating frequencies.

At block S42: obtaining the operating frequency corresponding to a maximum working voltage value of the piezoelectric pump and taking the operating frequency corresponding to the maximum working voltage value as an optimal operating frequency for a current time period.

At block S43: within a second time period after the first time period, setting the operating frequency of the piezoelectric pump to the optimal operating frequency and maintaining operation at the optimal operating frequency.

In any of the above embodiments, the adjusting the operating frequency of the piezoelectric pump within the first time period after the piezoelectric pump is started, and detecting the suction output capabilities of the piezoelectric pump at different operating frequencies include: adjusting the operating frequency of the piezoelectric pump within a preset frequency range during the first time period after the piezoelectric pump is started, and detecting the suction output capabilities of the piezoelectric pump at different operating frequencies.

In any of the above embodiments, the adaptive method further includes: detecting the operating frequency of the piezoelectric pump during operation of the breast pump, and determining a maximum value and a minimum value of the operating frequency; and determining the preset frequency range according to the maximum value and the minimum value.

In any of the above embodiments, the adaptive method further includes: readjusting the operating frequency of the piezoelectric pump within a third time period after the second time period, and detecting the suction output capabilities of the piezoelectric pump at different operating frequencies; obtaining the operating frequency corresponding to the maximum output capability of the piezoelectric pump and taking the operating frequency corresponding to the maximum output capability as the optimal operating frequency for the current time period; and setting the operating frequency of the piezoelectric pump to the optimal operating frequency and maintaining operation at the optimal operating frequency during a fourth time period after the third time period.

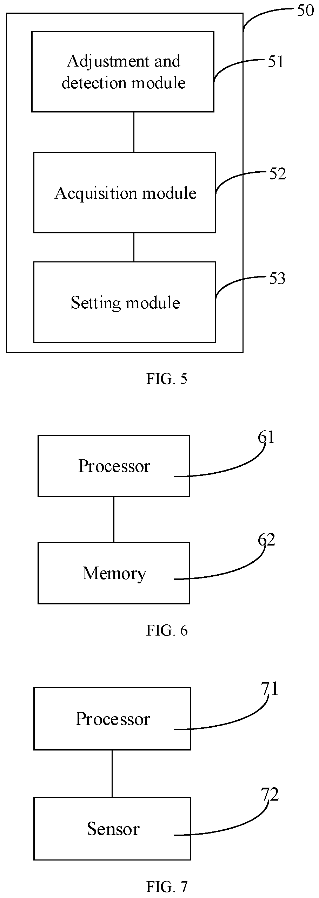

Referring to FIG. 5, FIG. 5 is a schematic block diagram of an adaptive apparatus for an operating frequency of a breast pump according to some embodiments of the present disclosure.

In the embodiments, the adaptive apparatus 50 for an operating frequency of a breast pump includes: an adjustment and detection module 51, an acquisition module 52, and a setting module 53.

The adjustment and detection module 51 is configured to, within a first time period after a piezoelectric pump of the breast pump is started, adjust an operating frequency of the piezoelectric pump, and detect suction output capabilities of the piezoelectric pump at different operating frequencies.

The acquisition module 52 is configured to obtain the operating frequency corresponding to a maximum output capability of the piezoelectric pump and take the operating frequency corresponding to the maximum output capability as an optimal operating frequency for a current time period.

The setting module 53 is configured to, within a second time period after the first time period, set the operating frequency of the piezoelectric pump to the optimal operating frequency and maintain operation at the optimal operating frequency.

Referring to FIG. 6, FIG. 6 is a schematic diagram of a hardware structure of a breast pump according to some embodiments of the present disclosure.

In the embodiments, a breast pump 60 includes a processor 61 and a memory 62 electrically connected to the processor 61. The memory 62 stores a computer program, and the processor 61 is configured to invoke the computer program to execute the adaptive method for an operating frequency of a breast pump according to any of the above embodiments.

Referring to FIG. 7, FIG. 7 is a schematic diagram of another hardware structure of a breast pump according to some embodiments of the present disclosure.

In the embodiments, the breast pump includes a processor 71 and a sensor 72 electrically connected to the processor 71. The processor 71 is configured to adjust an operating frequency of a piezoelectric pump of the breast pump within a first time period after the piezoelectric pump is started, and control the sensor 72 to detect suction output capabilities of the piezoelectric pump at different operating frequencies. The processor 71 is further configured to obtain the operating frequency corresponding to a maximum output capability of the piezoelectric pump and take the operating frequency corresponding to the maximum output capability as an optimal operating frequency for a current time period. The processor 71 is further configured to set the operating frequency of the piezoelectric pump to the optimal operating frequency and maintain operation at the optimal operating frequency within a second time period after the first time period.

In one implementation, the detecting the output capabilities of the piezoelectric pump at different operating frequencies is achieved by detecting a pressure value on a negative pressure output pipeline of the piezoelectric pump. That is, the sensor 72 may be a pressure sensor, and the pressure sensor is configured to detect output suction values of the piezoelectric pump at different operating frequencies. The pressure sensor may be installed at any position on the negative pressure output pipeline of the negative pressure pump, which is not limited in the embodiments of the present disclosure.

In one implementation, the detecting the output capability of the piezoelectric pump at different operating frequencies is achieved by detecting a working current value or working voltage of the piezoelectric pump. Correspondingly, the sensor 72 may be a voltage and current detection sensor, such as a current or voltage detection circuit. This detection circuit may be integrated in the processor or may be separately provided, which is not limited in the embodiments of the present disclosure.

In one implementation, the processor 71 keeps the working frequency of the negative pressure pump at the optimal operating frequency by sending the optimal operating frequency to a frequency locking circuit.

The frequency locking circuit may be a phase-locked loop circuit. The phase-locked loop circuit includes: 1. A voltage-controlled oscillator (VCO) for generating and outputting a drive signal related to the set target frequency to the piezoelectric pump. 2. A phase-frequency detector (PFD) for receiving the actual operating frequency signal of the piezoelectric pump and comparing it with the set optimal operating frequency signal to generate an error signal. 4. A low-pass filter (LPF) and a controller, which process the received error signal and then adjust the input voltage of the VCO, thereby changing the output frequency of the VCO. This phase-locked loop forms a closed-loop control system. Under the action of the phase-locked loop, the operating frequency of the piezoelectric pump automatically tracks and eventually locks onto the preset optimal operating frequency. Specifically, it may include the following steps: setting the target operating frequency of the phase-locked loop to the optimal operating frequency. The PFD calculates the error between the actual frequency and the target frequency and passes it to the LPF and the controller. The controller adjusts the output frequency of the VCO according to the error signal until the actual frequency matches the set optimal operating frequency, achieving a locked state. In the locked state, the system continuously monitors and dynamically fine-tunes to keep the operating frequency of the piezoelectric pump precisely locked to the set optimal operating frequency.

Embodiments of the present disclosure further provide a storage medium. The storage medium stores a computer program, and the computer program, when executed by a processor, can implement the adaptive method for an operating frequency of a breast pump according to any of the above embodiments.

The computer program may be stored in the aforementioned storage medium in the form of a software product, including several instructions to cause a device or processor to perform all or part of the steps of the methods described in the various embodiments of the present disclosure.

The storage medium is a medium in computer memory to store a certain discontinuous physical quantity. The aforementioned storage medium may be: a USB flash drive, a mobile hard disk, a read-only memory (ROM), a random-access memory (RAM), a magnetic disk, an optical disc, or various other media capable of storing program code.

In the several embodiments provided in the present disclosure, it should be understood that the disclosed methods and devices may be implemented in other ways. For example, the device embodiments described above are merely illustrative. For example, the division of modules or units is only a logical functional division. In actual implementation, there may be other division methods. For example, multiple units or components may be combined or integrated into another system, or some features may be omitted or not executed.

Units described as separate components may or may not be physically separate. Components shown as units may or may not be physical units, that is, they may be located in one place or distributed across multiple network units. Some or all of the units may be selected according to actual needs to achieve the objectives of the embodiments of the present disclosure.

Furthermore, the functional units in the various embodiments of the present disclosure may be integrated into one processing unit, or each unit may exist physically alone, or two or more units may be integrated into one unit. The above integrated units may be implemented in hardware form or in the form of software functional units.

The embodiments of the present disclosure, by adjusting the operating frequency of the piezoelectric pump within the first time period after the piezoelectric pump is started, and detecting the suction output capabilities of the piezoelectric pump at different operating frequencies; obtaining the operating frequency corresponding to the maximum output capability of the piezoelectric pump and taking the operating frequency corresponding to the maximum output capability as the optimal operating frequency for the current time period; and setting the operating frequency of the piezoelectric pump to the optimal operating frequency and maintaining operation at the optimal operating frequency during the second time period after the first time period, may make the piezoelectric pump operate in an optimal state, thereby improving the output power and efficiency of the breast pump's piezoelectric pump.

The above descriptions are only specific embodiments of the present disclosure. It should be pointed out that for those skilled in the art, several improvements and modifications may be made without departing from the principles of the present disclosure, and these improvements and modifications should be considered as within the scope of the present disclosure.

## Claims

1. An adaptive method for an operating frequency of a breast pump, comprising:
adjusting an operating frequency of a piezoelectric pump of the breast pump within a first time period after the piezoelectric pump is started, and detecting suction output capabilities of the piezoelectric pump at different operating frequencies;
obtaining the operating frequency corresponding to a maximum output capability of the piezoelectric pump and taking the operating frequency corresponding to the maximum output capability as an optimal operating frequency for a current time period; and
setting the operating frequency of the piezoelectric pump to the optimal operating frequency and maintaining operation at the optimal operating frequency within a second time period after the first time period.

2. The adaptive method according to claim 1, wherein the detecting suction output capabilities of the piezoelectric pump at different operating frequencies comprises:
detecting suction values output by the piezoelectric pump at different operating frequencies;
the obtaining the operating frequency corresponding to a maximum output capability of the piezoelectric pump and taking the operating frequency corresponding to the maximum output capability as an optimal operating frequency for a current time period comprises:
obtaining the operating frequency corresponding to a maximum suction value of the piezoelectric pump and taking the operating frequency corresponding to the maximum suction value as the optimal operating frequency for the current time period.

3. The adaptive method according to claim 1, wherein the detecting the suction output capabilities of the piezoelectric pump at different operating frequencies comprises:
detecting working current values of the piezoelectric pump at different operating frequencies;
the obtaining the operating frequency corresponding to a maximum output capability of the piezoelectric pump and taking the operating frequency corresponding to the maximum output capability as an optimal operating frequency for a current time period comprises:
obtaining the operating frequency corresponding to a maximum working current value of the piezoelectric pump and taking the operating frequency corresponding to the maximum working current value as the optimal operating frequency for the current time period.

4. The adaptive method according to claim 1, wherein the detecting the suction output capabilities of the piezoelectric pump at different operating frequencies comprises:
detecting working voltage values of the piezoelectric pump at different operating frequencies;
the obtaining the operating frequency corresponding to a maximum output capability of the piezoelectric pump and taking the operating frequency corresponding to the maximum output capability as an optimal operating frequency for a current time period comprises:
obtaining the operating frequency corresponding to a maximum working voltage value of the piezoelectric pump and taking the operating frequency corresponding to the maximum working voltage value as the optimal operating frequency for the current time period.

5. The adaptive method according to claim 1, wherein the adaptive method further comprises:
readjusting the operating frequency of the piezoelectric pump within a third time period after the second time period, and detecting the suction output capabilities of the piezoelectric pump at different operating frequencies;
obtaining the operating frequency corresponding to another maximum output capability of the piezoelectric pump and taking the operating frequency corresponding to the other maximum output capability as another optimal operating frequency for another current time period; and
setting the operating frequency of the piezoelectric pump to the other optimal operating frequency and maintaining operation at the other optimal operating frequency within a fourth time period after the third time period.

6. The adaptive method according to claim 1, wherein the adjusting an operating frequency of a piezoelectric pump of the breast pump within a first time period after the piezoelectric pump is started, and the detecting suction output capabilities of the piezoelectric pump at different operating frequencies comprise:
adjusting the operating frequency of the piezoelectric pump within a preset frequency range within the first time period after the piezoelectric pump is started, and detecting the suction output capabilities of the piezoelectric pump at different operating frequencies.

7. The adaptive method according to claim 6, wherein the adaptive method further comprises:
detecting the operating frequency of the piezoelectric pump during operation of the breast pump, and determining a maximum value and a minimum value of the operating frequency; and
determining the preset frequency range according to the maximum value and the minimum value.

8. A breast pump, comprising a processor and a memory electrically connected to the processor; wherein the memory stores a computer program, and the processor is configured to invoke the computer program to execute the adaptive method according to any one of claims 1-7.

9. An adaptive apparatus for an operating frequency of a breast pump, comprising:
an adjustment and detection module, configured to adjust an operating frequency of a piezoelectric pump of the breast pump within a first time period after the piezoelectric pump is started, and detect suction output capabilities of the piezoelectric pump at different operating frequencies;
an acquisition module, configured to obtain the operating frequency corresponding to a maximum output capability of the piezoelectric pump and take the operating frequency corresponding to the maximum output capability as an optimal operating frequency for a current time period; and
a setting module, configured to set the operating frequency of the piezoelectric pump to the optimal operating frequency and maintaining operation at the optimal operating frequency within a second time period after the first time period.

10. A storage medium, storing a computer program; wherein the computer program is capable of being executed to implement the adaptive method according to any one of claims 1-7.

11. A breast pump, comprising a processor and a sensor electrically connected to the processor; wherein the processor is configured to adjust an operating frequency of a piezoelectric pump of the breast pump within a first time period after the piezoelectric pump is started, and control the sensor to detect suction output capabilities of the piezoelectric pump at different operating frequencies;
the processor is further configured to obtain the operating frequency corresponding to a maximum output capability of the piezoelectric pump and take the operating frequency corresponding to the maximum output capability as an optimal operating frequency for a current time period; and
the processor is further configured to set the operating frequency of the piezoelectric pump to the optimal operating frequency and maintain operation at the optimal operating frequency within a second time period after the first time period.
